Europäisches Patentamt

European Patent Office

Office européen des brevets

(19)

(11) Publication number : **0 684 038 A2**

# EUROPEAN PATENT APPLICATION

(12)

(21) Application number : 95301808.2

(22) Date of filing : 17.03.95

(51) Int. Cl.$^6$ : **A61K 7/32**

(30) Priority : 31.03.94 GB 9406461

(43) Date of publication of application :
29.11.95 Bulletin 95/48

(84) Designated Contracting States :
CH DE ES FR GB IT LI NL SE

(71) Applicant : UNILEVER PLC
Unilever House
Blackfriars
London EC4P 4BQ (GB)
(84) GB

(71) Applicant : UNILEVER N.V.
Weena 455
NL-3013 AL Rotterdam (NL)
(84) CH DE ES FR IT LI NL SE

(72) Inventor : Correia, Martinho
4 Ashburn Close
Wetherby, West Yorkshire, LS22 5RB (GB)
Inventor : Timme, Susan Mary
16 The Burrows
Birstall, West Yorkshire, WF17 8BE (GB)
Inventor : Wight, Gordon R., Unilever Research
Port Sunlight Lab.,
Quarry Road East,
Bebington
Wirral, Merseyside, L63 3JW (GB)

(74) Representative : Gordon, Naoise Padhraic
Edward et al
Unilever plc
Patent Division
Colworth House
Sharnbrook
GB-Bedford MK44 1LQ (GB)

(54) Deodorant compositions.

(57)   The invention provides a propellant driven alcoholic aerosol composition for topical application to the human skin, comprising a deodorant agent which comprises a short chain monohydric alcohol, a propellant, a perfume, and 0.01-0.3% by weight of a thickening agent comprising a natural gum derivative, a synthetic cellulose derivative gum or a carboxyl vinyl polymer.

EP 0 684 038 A2

This invention relates to aerosol deodorant products. In particular, it relates to thickened aerosol deodorant products, for topical application to the human skin.

The market in aerosol deodorant products is dominated by propellant driven aerosol products, which are often based on a relatively simple combination of a deodorant agent (which in many cases is simply a short chain monohydric alcohol such as ethanol), together with a propellant and a perfume. Such compositions may typically additionally comprise various commonly used cosmetic adjuncts, such as skin feel improving agents, emollients, humectants, and antimicrobial compounds, to name but a few.

Such conventional compositions typically have many problems associated with them, of differing natures. In particular, a commonly encountered problem with deodorant compositions is how to improve the efficacy, in particular over a relatively long time frame (for example 8 hours or longer after application). This is particularly the case when the deodorant agent comprises only a short chain monohydric alcohol.

It is an object of the invention to overcome problems associated with conventional deodorant compositions, and in particular to provide a deodorant composition with improved longer term efficacy.

Thus, according to one aspect of the invention, there is provided a propellant driven alcoholic aerosol deodorant composition for topical application to the human skin, comprising a deodorant agent which comprises a short chain monohydric alcohol, a propellant, a perfume, and a thickening agent comprising a natural gum derivative, a synthetic cellulose derivative gum, or a carboxyl vinyl polymer.

The thickening agent used in compositions according to the invention is conveniently a natural gum derivative such as xanthan gum, tragacanth, gum acacia, or locust bean gum, or a synthetic cellulose-based gum derivative such as hydroxymethyl cellulose, hydroxypropyl cellulose, methyl cellulose or carboxymethyl cellulose, or a carboxyl vinyl polymer such as a carbomer, sold by Goodrich under the trade name Carbopol.

A highly preferred thickening agent is a hydroxypropyl cellulose derive, in particular Klucel. Particularly preferred Klucel materials, which are made by Hercules include Klucel M. In particular when the thickening agent is a hydroxypropyl cellulose derivative, it is highly preferred that the material has a molecular weight of greater than $2 \times 10^5$, since it has been found that by using a thickening agent with such a high molecular weight, only a very small amount of the thickening agent is required to provide a substantial increase in capture efficiency of the spray product in use. The thickening agent is preferably found in the composition at a level of 0.01-0.3% by weight of the composition, more preferably 0.05-0.1% by weight of the composition.

According to another aspect of the invention, there is provided a propellant driven alcoholic aerosol deodorant composition for topical application to the human skin, consisting essentially of a deodorant agent which comprises a short chain monohydric alcohol, a propellant, a perfume, and a thickening agent comprising a natural gum derivative, a synthetic cellulose derivative gum or a carboxyl vinyl polymer.

According to a further aspect of the invention, there is provided a method of applying a topical alcoholic aerosol deodorant composition to the skin, comprising providing a pressurised composition comprising a deodorant agent which comprises a short chain monohydric alcohol, propellant, a perfume, and a thickening agent comprising a natural gum derivative, a synthetic cellulose derivative gum or a carboxyl vinyl polymer in a pressurised container, and applying the pressurised composition to the human skin.

According to yet a further aspect of the invention, there is provided the use of a thickening agent comprising a natural gum derivative or a synthetic cellulose derivative gum in a propellant driven alcoholic aerosol deodorant composition for topical application to the human skin, for the purpose of improving the long term efficacy of the deodorant composition.

By "long term" in this instance is meant at a period of twenty four hours after application, or even longer.

It is surprisingly been found that propellant driven alcoholic aerosol deodorant compositions, in particular deodorant aerosol compositions which contain a short chain (C2-C6, preferably C2) monohydric alcohol, have been found to have superior long term efficacy to similar compositions not containing the defined thickening agent. In particular, it is considered surprising that suitable aerosol deodorant compositions containing such a natural gum derivative or synthetic cellulose derivative gum or carboxyl vinyl polymer thickening agent can be manufactured, since it was thought that the inclusion of such a thickening agent into the aerosol deodorant composition would cause it to be too thick to use as to spray, and also cause nozzle blockage problems. This has been found not to be the case.

## The Propellant

Preferably, the propellant used in compositions according to the invention is a gaseous propellant. Suitable propellants which are normally liquefiable, include propane, n-butane, iso-butane, dimethylether and other hydrocarbon propellants. The propellant gases may be used individually or in admixture, and with or without any other volatile liquids.

Examples of propellants which are not normally liquefiable under conditions of normal ambient use include carbon dioxide, nitrogen, nitrogen oxides and air, with these normally being used in the compressed state in conjunction with a suitable aerosol container.

The amount of propellant in the packaged composition can be 0.1-99%, but more typically is 50-99% of the composition, more preferably 55-95% by weight of the composition.

## The Deodorant Agent

The deodorant agent used in compositions according to the invention may be any deodorant agent which would readily be used by the skilled man in a topically applicable deodorant composition. The deodorant agent may be organic or inorganic, and essentially comprises at least one short chain monohydric alcohol, in particular ethanol. Preferably the composition comprises at least 5% short chain monohydric alcohol.

The composition may also additionally comprise another organic deodorant agent; in this context, preferred deodorant agents include citricidal and usnic acid derivatives (e.g. Deousnate, ex Cosmetochem). However others, such as triclosan or other antimicrobials may be used.

Alternatively, the deodorant agent may be an inorganic deodorant active. Such deodorant agents may typically be metal salts, such as those based on aluminium, zirconium, zinc or mixtures thereof. A preferred inorganic deodorant agent is zinc phenolsulphonate.

Mixtures of organic and inorganic deodorant agents are contemplated by the invention.

Preferably, the deodorant agent is present in the composition at levels of 0.1-80% by weight of the composition, of which the short chain monohydric alcohol may constitute most or all of the deodorant agent. Other deodorant agents used in the composition (excluding the short chain monohydric alcohol) may be present at a level of up to 10% by weight of the composition.

## The Perfume

The composition according to the invention additionally comprises of perfume. This may be any conventional perfume, such as a perfume oil, but also includes so called deoperfumes, which may provide their own deodorant effect. These are further described in for example, EP 5,618, GB 2,016,507, EP 545,556, GB 2,013,493, and EP 404,470, the contents of which are incorporated herein by reference. Also contemplated are compositions which when commercially sold are labelled "unscented", which contain only a minor amount of perfume which is sufficient to mask any base odour in the composition. Preferably, the perfume is present in the composition at a level of 0.01-3% more preferably 0.01-2% by weight of the composition.

## Thickening Agent

Compositions according to the invention comprise a thickening agent which comprises a natural gum derivative or a synthetic cellulose derivative gum. Preferably, the thickening agent is a hydroxypropyl cellulose gum, and more preferably is Klucel M, ex. Hercules. Preferred hydroxypropyl cellulose thickening agents for use in compositions according to the invention have a molecular weight of at least $2 \times 10^5$. Preferably, the thickening agent is used in compositions according to the invention at a level of 0.01-0.3% by weight of the composition, preferably 0.05-0.1% by weight of the composition. Such levels are often insufficient to provide any suspending effect of particles or thickening in the composition, but do nevertheless lead to benefits in use in terms of the improved deodorant efficacy observed.

Without wishing to be bound by theory, it is though possible that such low levels of relatively high molecular weight thickeners in the aerosol composition act so as to increase the droplet size of the spray in use. They may also act to otherwise inhibit evaporation of the deodorant agents in the composition in use, in particular the volatile deodorant agents such as short chain monohydric alcohols, and thereby maximise retention of deodorant agents on the skin.

## Cosmetic Adjuncts

The composition may additionally comprise other components typically found in deodorant compositions for topical application to the skin, including;
- linear or cyclic volatile silicones, including polydimethyl siloxanes such as Dow Corning 344 Fluid and Dow Corning 345 Fluid, and volatile hexamethyldisiloxane such as Dow Corning 200 Fluid,
- non-volatile silicones, including polydimethylsiloxane such as Dow Corning Fluids,
- skin feel improvers, such as talc and finely divided polyethylene, for example Acumist B18,
- cosmetically acceptable vehicles, such as water, glycols such as propylene glycol or butylene glycol, and emollients,
- gelling agents, such as stearyl alcohol, sodium stearate or waxes, such as castor wax,
- preservatives, and
- other cosmetically acceptable adjuncts conventionally employed in propellant driven spray deodorant products.

## EXAMPLES

The invention will now be described by way of example only.

Test Protocol

In this test the deodorant value of a deodorant composition is measured by assessing its effectiveness when applied directly to the skin. The composition is applied to the axillae (armpits) of a panel of human subjects, and its effect at reducing malodour is subsequently assessed.

The test uses a team of 3 or 4 experienced female under-arm odour assessors. These are selected on the basis that they are able to rank correctly the odour intensities of a series of isovaleric acid solutions listed below. The intensities of this series of solutions forms the basis of the 0-5 category scale used to quantify under-arm odour subjectively in the test.

Malodour Category Scale

| SCORE | ODOUR LEVEL | AQUEOUS ISOVALERIC ACID CONC, ml/litre |
|-------|-------------|------------------------------------------|
| 0 | no odour | 0 |
| 1 | slight | 0.013 |
| 2 | definite | 0.053 |
| 3 | moderate | 0.220 |
| 4 | strong | 0.870 |
| 5 | very strong | 3.57 |

New assessors are trained by participation alongside experienced assessors in the tests of deodorancy of underarm products. Training is complete when the new assessor is able to quantify underarm odour in the present protocol on the same basis as experienced assessors. Discrimination between underarm odour and residual product perfume in the total axillary odour is a key part of the skill of an expert odour assessor.

The panel consists of up to 50 subjects who have been selected by the expert odour assessors on the basis that their body odour is not unusually weak or strong, or uneven between axillae. The panellists are denied the use of any underarm products, and are provided with a non-deodorant soap bar for home use.

The test is run over a five day period, and includes 4 product application-odour assessment cycles. The period between product application to subjects and the assessment of under-arm odour is 24 hours. Only panellists who have not participated in any testing of under-arm products for at least a week previously can take part in this test, because product carry-over effects can occur.

On the first day of the test, the panellist's axillae are washed by the assessors using unperfumed soap. A standard technique is used in which a wet flannel is soaped for 15 seconds, the axilla washed with the flannel for 30 seconds, then wiped with a water-rinsed flannel and dried with a paper towel. A separate flannel is used for each axilla of each panellist. Each panellist wears a different product in each axilla and this is kept constant throughout the test.

A standard product application technique is used; for aerosols, a timed 2 second spray, 6 inches from the axilla is used; after 24 hours wear of the test product the odour intensity of each axilla is evaluated by each assessor. Each in turn assigns a score on the scale of 0 to 5, corresponding to the strength of the under-arm malodour.

After 24 hours of the test, panellists are first evaluated for under-arm odour; then they are washed and the products re-applied. The protocol is repeated over 4 consecutive days. On the last day of the test panellists are assessed but no washing is carried out and no further product is applied.

Scores from the 4 day's assessments for all expert odour assessors are averaged to give a result for each test product for the week, and these are then averaged to give a team score. The results are analyses using a SAS-based Analysis of Variance routine which takes into account the factors which lead to variability, e.g. subject, day, left/right bias. The analysis also gives the difference in malodour score needed for a result to be statistically significant at the p=0.05 level. The deodorant value accorded to a test composition is the difference in score between the test composition and a placebo.

This test for determining the deodorant value of a deodorant composition for use in accordance with the invention is generally based on the test devised by Whitehouse and Carter as published in 'The Proceedings of the Scientific Section of the Toilet Goods Association", No. 48, December 1967 at pages 31-37 under the title "Evaluation of Deodorant Toilet Bars".

The test described in that publication has however generally been modified in two main ways: firstly, the product is applied directly to the axillae rather than by way of a soap bar wash, and secondly, a 0 to 5 instead of a 0 to 10 grading scale was employed.

Compositions

The following compositions were prepared and applied to the human volunteer panel, according to the above protocol.

| COMPOSITION | | | | |
|---|---|---|---|---|
| | 1 | 2 | 3 | 4 |
| Ethanol | 47.5 | 46.5 | 47.425 | 47.175 |
| Perfume | 1.50 | 1.50 | 1.50 | 1.50 |
| Klucel | - | - | 0.075 | 0.075 |
| Citricidal | - | 1.0 | - | 0.25 |
| Propellant | Balance ----------------------------------------------> | | | |

| COMPOSITION | | | | |
|---|---|---|---|---|
| | 5 | 6 | 7 | 8 |
| Ethanol | 47.425 | 46.825 | 47.125 | 47.275 |
| Perfume | 1.50 | 1.50 | 1.50 | 1.50 |
| Klucel | 0.075 | 0.075 | 0.075 | 0.075 |
| Deousnate | - | 0.6 | 0.3 | 0.15 |
| Propellant | Balance ----------------------------------------------> | | | |

* Composition 1 acted as a control for the test of compositions 2-4, and 5 for 6-8.

Note all compositions additionally comprised 1% isopropyl myristate as an emollient.

## RESULTS

| Composition | % Odour Reduction* |
|:-----------:|:------------------:|
| 2 | 6 |
| 3 | 16 |
| 4 | 21 |
| 6 | 7 |
| 7 | 5 |
| 8 | 7 |

* As measured against the control.

The results clearly show a benefit in efficacy as measured at 24 hours for those compositions containing the cellulose derivative thickening agent, compared to those compositions without it. The benefit is clear both in compositions which contain ethanol as the sole deodorant agent (e.g. example 3), and also those where there are further deodorant agents present (e.g. examples 4, 6-8). Moreover, the presence of the thickening agent and the deodorant agent (e.g. composition 4) provided a higher percentage odour reduction than those compositions containing either the deodorant agent alone (e.g. composition 2) or the thickening agent alone (e.g. composition 3).

Further experiments carried out indicated that a given composition containing 0.01% Klycel M (which has a molecular weight of $6.5 \times 10^5$) had a superior capture efficiency in use to a composition not containing this amount of Klucel M. A similar composition containing as a thickener Luviskol K90, a polyvinyl pyrrolidone thickener, ex BASF, required 2% of the thickener to be added to provide an equivalent benefit, in terms of improved capture efficiency, to the composition containing 0.01% Klucel M.

## Claims

1.   A propellant driven alcoholic aerosol composition for topical application to the human skin, comprising a deodorant agent which comprises a short chain monohydric alcohol, a propellant, a perfume, and 0.01-0.3% by weight of a thickening agent comprising a natural gum derivative, a synthetic cellulose derivative gum or a carboxyl vinyl polymer.

2.   An aerosol composition according to claim 1, wherein the thickening agent comprises a natural gum derivature comprising xanthan gum, tragacanth, gum acacia, or locust bean gum, or mixtures thereof.

3.   An aerosol composition according to claim 1, wherein the thickening agent comprises a synthetic cellulose based gum which comprises hydroxymethyl cellulose, hydroxy propyl cellulose, methyl cellulose, or carboxymethyl cellulose, or mixtures thereof.

4.   An aerosol composition according to claim 1, wherein the carboxy vinyl polymer is a carbomer.

5.   An aerosol composition according to any of the preceding claims, comprising at least 5% by weight of a short chain monohydric alcohol.

6.   An aerosol composition according to any of the preceding claims, wherein the deodorant agent is inorganic, and comprises a deodorant active salt of aluminium, zirconium, or zinc.

7.   An aerosol composition according to any of the preceding claims, wherein the thickening agent has a molecular weight of at least $2 \times 10^5$.

6